Europäisches Patentamt

European Patent Office

Office européen des brevets

(19)

(11) Publication number: **0 388 245**

**A1**

## (12) EUROPEAN PATENT APPLICATION

(21) Application number: 90400272.2

(51) Int. Cl.⁵: **A61K 33/42, A61K 33/24**

(22) Date of filing: **01.02.90**

The title of the invention has been amended (Guidelines for Examination in the EPO, A-III, 7.3).

(30) Priority: **01.02.89 JP 23170/89**

(43) Date of publication of application:
**19.09.90 Bulletin 90/38**

(84) Designated Contracting States:
**BE CH DE FR GB IT LI SE**

(71) Applicant: **Terumo Kabushiki Kaisha**
**No. 44-1, Hatagaya 2-chome Shibuya-ku**
**Tokyo 151(JP)**

Applicant: **Fujita, Haruhisa**
**11-8, Shoudo 2-chome, Sakae-ku**
**Yokohama-shi, Kanagawa-ken(JP)**

Applicant: **Yamase, Toshihiro**
**13-905, Wakabadai 4-chome, Asahi-ku**
**Yokohama-shi, Kanagawa-ken(JP)**

Applicant: **Fukushima, Kouji**
**23-5-3, Hijirigaoka 2-chome**
**Tama-shi, Tokyo(JP)**

Applicant: **Seto, Yoshiko**
**2-3-406, Natsumidai 1-chome**
**Funabashi-shi, Chiba-ken(JP)**

(72) Inventor: **Nakamura, Shoshiro**
**12-2, Koi-Osako 2-chome, Nishi-ku**
**Hiroshima-shi, Hiroshima-ken(JP)**
Inventor: **Fujita, Haruhisa**
**11-8, Shoudo 2-chome, Sakae-ku**
**Yokohama-shi, Kanagawa-ken(JP)**
Inventor: **Yamase, Toshihiro**
**13-905, Wakabadai 4-chome, Asahi-ku**
**Yokohama-shi, Kanagawa-ken(JP)**
Inventor: **Fukushima, Kouji**
**23-5-3, Higirigaoka 2-chome**
**Tama-shi, Tokyo(JP)**
Inventor: **Seto, Yoshiko**
**2-3-406, 1-chome, Natsumidai 1-chome**
**Funabashi-shi, Chiba-ken(JP)**

(74) Representative: **Gillard, Marie-Louise et al**
**Cabinet Beau de Loménie 55, Rue**
**d'Amsterdam**
**F-75008 Paris(FR)**

(54) **Agent resistant to AIDS virus.**

(57) An agent active against AIDS virus, having as an effective component thereof a salt of a heteropoly acid ion represented by the following general formula I:

$$[XM_{12}O_{40}]^{p-} \quad (I)$$

wherein X is an ion of one element selected from the group consisting of the elements of Groups III to VI in the Periodic Table of Elements and the transition metal elements, M is one ion or a mixture of up to 3 ions selected from the group consisting of the ions of molybdenum, tungsten, aluminum, vanadium, niobium, tantalum, cobalt, nickel, and titanium, O for an oxygen atom, and p is a positive integer.

## AGENT RESISTANT TO ACQUIRED IMMUNO DEFICIENCY SYNDROME VIRUS

## BACKGROUND OF THE INVENTION

Field of the Invention:

This invention relates to an agent resistant to the acquired immuno deficiency syndrome virus, having as an effective component thereof a heteropoly acid salt exhibiting an antiviral activity and manifesting usefulness as a medicine.

Description of the Prior Art:

The term "acquired immuno deficiency syndrome" refers to a disease which is prevalent among narcotic addicts and male homosexuals. Further, the infection of this disease through the medium of blood preparations has been posing a problem. Generally, this disease is called AIDS (acquired immuno deficiency syndrome). When a person contracts this disease, he develops conspicuous deficiency in immuno function and dies of an infectious disease. The fatality of patients of this disease is extremely high.

Various theories have been proposed regarding the mechanism of development of AIDS. It is considered, for example, that immunodeficiency is induced because the AIDS virus (HIV) successively infects the CD-4 antigen positive helper T cells as target cells and repeats viral hyperplasia and cellular fracture. Concerning the mechanism of development of AIDS, many points remain yet to be elucidated.

Studies of molecular biology in this field have been advancing remarkably and have been gradually unveiling the characteristics of HIV. It is plain that the HIV is characterized by possessing a conspicuously intricate genic structure as compared with other retroviruses, exhibiting versatility even in such functions as infecting property and hyperplastic ability, and showing a tendency to undergo variation, for example.

The knowledges of this nature have ushered in proposition of methods for inhibiting hyperplasia of AIDS virus and method for preventing infection of AIDS virus. It is nucleic acid derivatives and reverse transcriptase inhibitors that predominate over other substances for inhibiting hyperplasia of AIDS virus. Most of them were developed and put to use fairly long ago for other purposes. It is AZT that has been studied most extensively to date. Even in a freshly infected system of MT-4 cells, the AZT selectively suppresses the hyperplasia of the HIV. Even in a clinical test, the AZT has been demonstrated as effective in a control experiment using placebo for comparison. It has been known that the AZT, during the course of a protracted administration, brings about a secondary effect of inflicting injury to the bone marrow. The development of a safe medicine which takes the place of the AZT, therefore, constitutes an urgent task.

At present, medicines effective in combating the AIDS virus have been developed and put to actual use. None of the agents resistant to AIDS virus so far developed has demonstrated sufficient efficacy. The insufficient effect manifested in the therapy of AIDS and the secondary effect entailed by the administration of medicine persist as unsolved problems. Thus, the desirability of perfecting a medicine capable of solving these problems and taking the place of the existing medicines has been finding widespread recognition.

An object of this invention, therefore, is to provide a novel agent resistant to AIDS virus which possesses the nature fit for satisfying the desirability. Another object of this invention is to provide an agent resistant to AIDS virus which possesses a very strong action of resisting the AIDS virus and induces a very weak secondary effect.

## SUMMARY OF THE INVENTION

The objects described above are accomplished by an agent resistant to AIDS virus, having as an effective component thereof a salt of a heteropoly acid ion represented by the following general formula I:

$$[XM_{12}O_{40}]^{p-} \quad (I)$$

wherein X is an ion of one element selected from the group consisting of the elements of Groups III to VI in the Periodic Table of Elements and the transition metal elements, M is one ion or a mixture of up to 3 ions selected from the group consisting of the ions of molybdenum, tungsten, aluminum, vanadium, niobium, tantalum, cobalt, nickel, and titanium, O is an oxygen atom, and p is a positive integer.

When the agent resistant to the AIDS virus of this invention is prepared in the form of an aqueous solution, it exhibits a pH value in the range of 4 to 8 and enjoys a sufficiently high chemical stability.

The expression "sufficient chemical stability" as used herein represents the fact that the change which occurs in the aqueous solution of a poly acid compound owing to the aging at normal room temperature, as determined by the measurement of electric current generated by electrochemical reduction of a corresponding polyacid ion, is substantially nil. Specifically, this expression represents the condition of the polyacid compound of this invention such that when aqueous solutions containing 0.1 mol of the poly acid compound and having the pH value adjusted to varying levels in the range of 4 to 8 (the adjustment effected by the use of $HClO_4$, HCl, and other buffers) are subjected to a derivative polarographic test (by the use of a polarograph produced by EC & G and marketed under product code of "PRA-1744303") using a dropping mercury electrode, the peak value of the electric current of the first reduction wave (the reduction wave showing the most positive potential) of the polyacid ion is retained at or above 70% at a measuring temperature of 2.5oC for a period of one hour following the start of the measurement.

The salts of the present invention may be obtained by the processes disclosed in following examples 1 or 2, or by any equivalent processes thereof well known by the one skilled in the art.

The objects described above are also accomplished by an medicinal composition resistant to AIDS virus, comprising a salt of a heteropoly acid ion represented by the following general formula I:

$$[XM_{12}O_{40}]^{p-} \quad (I)$$

wherein X and M have the same meanings as defined above, and a pharmaceutically acceptable carrier.

These objects are further accomplished by the use of a salt of a heteropoly acid ion represented by the following general formula I:

$$[XM_{12}O_{40}]^{p-} \quad (I)$$

wherein X and M have the same meanings as defined above for the preparation of a medicinal composition useful for the treatment of AIDS.

## EXPLANATION OF THE PREFERRED EMBODIMENT

The pentapotassium dodecatungstoborate and heptapotassium dititanate decatungstophosphate which are severally used in the agent of this invention resistant to AIDS are salts of Keggin form heteropoly acid ions represented by the general formula:

$$[XM_{12}O_{40}]^{p-}$$

wherein X is an ion of one element selected from the group consisting of the elements of Groups III to VI in the Periodic Table of Elements and the transition metal elements, M is one ion or a mixture of up to 3 ions selected from the group consisting of the ions of molybdenum, tungsten, aluminum, vanadium, niobium, tantalum, cobalt, nickel, and titanium, O is an oxygen atom, and p is a positive integer or heteropoly acid ion.

The polytungstates to be used in the present invention are heteropoly acids of one kind which are cluster compounds, namely oxy acid ion polynuclear complexes so configured that basic units having generally four or six oxygen atoms attached to a tungsten atom are bound to one another through the medium of edges or apexes. The poly acid ions in such a heteropoly acid are generally characterized as follows.

(1) The ion size is in the range of 6 to 25 Å and the molecular weight in the range of 1,000 to 10,000.

(2) The ion is mostly obtained in the form of a catalyst and exhibits high solubility to water and polar solvents.

(3) The number of molecules of hydration is large.

(4) The ion functions as a strong oxidizing agent (polyelectron-pooling agent).

The poly acid ion in solid or solution possesses the nature of effecting a photo-oxidation reduction reaction and is expected to find extensive commercial utility in application to display devices. Since these compounds possess high reactivity, they are expected to exhibit physiological activity. As the result of various studies, it has been found that poly acid ions such as, for example, polytungstates possess a very conspicuous antiviral effect, particularly a medicinal effect on AIDS virus and other human retroviruses which have been posing a serious problem in recent years. This fact is predictable from the observation that these polytungstates possess an activity to inhibit the activity of reverse transcriptase of viruses.

The polytungstates of this description include salts of cations such as sodium, potassium, cesium, calcium, strontium, barium, ammonium, hydrogen, methyl ammonium, ethyl ammonium, dimethyl ammonium, trimethyl ammonium, tetramethyl ammonium, diethyl ammonium, isopropyl ammonium, diisopropyl ammonium, propyl ammonium, dipropyl ammonium, butyl ammonium, dibutyl ammonium, tributyl ammonium, tetrabutyl ammonium, and guanidinium, for example.

The following compounds may be cited as examples of the heteropoly acid salt of this invention.

1. $[XW_{12}O_{40}]^{p-}$: X = H, $H_2$, B, Al, Ga (III), P(V), As(V), Cr(III), Mn(IV), Fe(III), Co(III), Co(II), Cu(II), Cu(I), Zn, Se(IV), Te(IV), Sb(III), Bi(III).

2. $[XMo_{12}O_{40}]^{p-}$: X = Si, Ge(IV), P(V), As(V), V(v), Ti(IV), Zn(IV), In(III), $H_2$, Mo.

3. $[PVMo_{12-x}O_{40}]^{(3+x)-}$: X = 1~3;
$[PVW_{12-x}O_{40}]^{(3+x)-}$: X = 1~4;

4. $[ZnW_{11}CoO_{40}]9-$,
$[ZnW_{11}CoO_{40}]^{10-}$,
$[GaW_{11}CuO_{40}]^{10-}$,
$[GaW_{11}GaO_{40}]^{8-}$,
$[Pw_{10}Ti_2O_{40}]^{7-}$,
$[Pw_{11}TiO_{40}]^{6-}$.

5. $[(n-C_4H_9)_4N]_4 SiMo_{12}O_{40}$,
$H_4 GeMo_{12}O_{40}$,
$Na_4 GeMo_{12}O_{40}$,
$[(n-C_4H_9)_4N]_4 GeMo_{12}O_{40}$,
$Na_2HPMo_{12}O_{40}$,
$[(n-C_4H_9)_4N]_4 PMo_{12}O_{40}$,
$[(n-C_4H_9)_4N]_4 AsMo_{12}O_{40}$,
$K_5 BW_{12}O_{40}$,
$[(n-C_4H_9)_4N]_4 HBW_{12}O_{40}$,
$H_4 SiW_{12}O_{40}$,
$K_4 SiW_{12}O_{40}$,
$Ba_2 SiW_{12}O_{40}$,
$[(CH_3)_4N]_4 SiW_{12}O_{40}$,
$[(n-C_4H_9)_4N]_4 SiW_{12}O_{40}$,
$(NH_4) SiW_{12}O_{40}$,
$H_4 GeW_{12}O40$,
$K_4 GeW_{12}O_{40}$,
$[(n-C_4H_9)_4N]_4 GeW_{12}O_{40}$,
$H_3 PW_{12}O_{40}$,
$Na_3 PW_{12}O_{40}$,
$Na_3 AsW_{12}O_{40}$,
$[(n-C_4H_9)_4N]_4 AsW_{12}O_{40}$,
$K_6 [SiNiW_{11}O_{40}H_2]$.

The agent resistant to the AIDS virus in accordance with this invention generally contains in a solid or solution carrier, preferably a liquid carrier, at least one compound represented by the general formula $[XM_{12}O_{40}]^{p-}$. The substances which are usable as the carrier herein include any pharmaceutically acceptable carrier well known by the one skilled in the art. The compounds of this invention may be used in combination with known active substances. The agent of this invention may be administered for antiviral purpose in any of suitable and/or conventional manners. When the compound is administered through a non-oral, hypodermic, intravenous, or intraabdominal route, the carrier for the compound is an abacterial liquid such as distilled water or physiological saline solution, preferably water. The aqueous carrier in the aqueous solution for injection contains the effective substance in a ratio of 3 to 12 mg/ml, for example 10 mg/ml (1.0%). The compound according to this invention can be administered in a form fit for oral administration. It may be used advantageously in the form of ointment or eye lotion, for example. Other forms in which the compound can be orally administered preferably include tablets, capsules, powder, and suspension, for example. In the compositions prepared for administration in these forms, the content of this compound is desired to be in the range of 1.0 to 3.0% by weight, based on the total amount of the composition. The dosage of this compound for adult per day is in the range of 100 to 3,000 mg.

Now, this invention will be described specifically below with reference to working examples.

Synthesis 1

4

In a glass beaker, 100 g of sodium tungstate and 5 g of boric acid were dissolved in 100 ml of purified water and the resultant aqueous solution was adjusted to pH 2.0 by addition of 6N hydrochloric acid. This acidic solution was refluxed for about 5 hours and cooled. The resultant condensate was adjusted to a solution pH of about 2.0 with hydrochloric acid, refluxed for 30 minutes, mixed with 20 g of potasium chloride, and cooled. The precipitate consequently formed in the cooled solution was separated by filtration, washed twice with diethyl ether, and recrystallized twice with hot water. By elementary analysis, thermal analysis, and NMR spectrum, the crystals consequently obtained were identified to be those of $K_5BW_{12}O_{40} \cdot 15H_2O$.

Synthesis 2

In a glass flask, 6 g of sodium dihydrogen phosphate and 3og of sodium tungstate were dissolved in 100 ml of purified water. The resultant aqueous solution was kept stirred and 1.8 ml of titanium tetrachloride was slowly added dropwise thereto. The solution was refluxed for 20 minutes and immediately filtered. The filtrate was mixed with 30 g of potassium chloride. The precipitate consequently formed therein was separated by filtration and recrystallized twice from hot water. By elementary analysis, thermal analysis, and NMR spectrum, the crystals thus obtained were identified to be those of $K_7PW_{10}Ti_2O_{40} \cdot 6H_2O$ (Compound A).

Example 1

Test for activity to inhibit hyperplasia of antihuman immunodeficiency virus (HIV)

A test for the activity to inhibit hyperplasia of HIV was conducted in accordance with the method proposed by Harada et al [Science, 229, 563-5663 (1985)].

Testing conditions:

Cell: MT-in (HTLV-I-carrying cell line)

Virus: THLV-IIIb (one kind of HIV); HTLV-IIIb was prepared from the supernatant of the culture broth of MOLT-4/HIV, a HIV-producing cell. The potency of the virus was $1 \times 10^5$ TCID 50/ml.

Culture medium: RPMI-1640 (containing 10% bovine fetal serum, 100 IV/penicilin, and 100 $\mu g/ml$ streptomycin)

Culture conditions: In each of the wells of a 24-well culture tray, MT-in cells ($6.6 \times 10^5$/ml, 0.3 ml) and HTLV-II Ib (2 x TCID 50/ml, 0.2 ml) were placed [multiplicity of infection (MOI = 0.02)] and cultured for 1 hour. After adsorption of virus, Compound A diluted with culture medium (0.5 ml) was added to a total volume of 1.0 ml and cultured at 37°C for 4 days. Cells not infected by HTLV-IIIb dilution aqueous solution and cells not treated with Compound A were used as controls.

Test for cytopathix effect (CPE):

Virally infected cells and uninfected cells were cultured for 4 days. After the culture, the live cells were counted by the trypan blue tinting method. The property of cytopathic effect suppression was evaluated by comparison of the number of live cells in the culture broths of virally infected cells and uninfected cells both obtained in the absence of Compound A.

The results of the anti-HIV activity (CPE) were shown in Table 1.

Table 1

| | | | | | (x $10^5$ cells/ml) | |
|---|---|---|---|---|---|---|
| Compound A (μg/ml) | 400 | 200 | 100 | 50 | 25 | None used |
| MT-4 | 6.0 | 8.5 | 10.1 | 9.8 | 11.8 | 10.8 |
| MT-4/HIV | 4.7 | 4.8 | 8.5 | 9.8 | 10.0 | 2.8 |
| *1 | 44 | 21 | 0 | 1 | 0 | - |
| *2 | 47 | 25 | 71 | 88 | 90 | - |

*1: Toxicity (% control)
*2: % CPE Control, (MT-4/HIV-28)/(108-28) × 100
MT-4: HTLV-1 infection T-4 antigen positive ATL cells

It is clearly noted from Table 1, particularly from comparison between MT-4 and (mT-4/HIV), that the compound A at a rate in the range of 25 to 100 ug/ml showed only inconspicuous toxicity and excelled in activity to control CPE.

Example 2

Test for activity to inhibit reverse transcriptase:

This test was carried out by the method described below.

| Composition of measuring solution | |
|---|---|
| Tris-hydrochloric acid buffer (pH 8.0) | 100 mM |
| MgCl₂ | 5mM |
| NaCl | 60mM |
| Dithiothreitol | 5mM |
| [³H] thymidine triphosphoric acid ; 12.5 μ Ci/ml | 0.2mM |
| Polyadenylic acid | 10 μg/mℓ |
| Deoxythimidylic acid oligomer (12 to 18) | 0.04U/mℓ |
| Reverse transcriptase originating in avain myeloblastic virum (produced by Seikagaku Kogyo K.K.) | 9.0U/mℓ |

Test solution:

This test solution was prepared by dissolving Compound A in a concentration of 5 mg/ml in dimethyl sulfoxide and diluting the resultant solution with distilled water.

Method of measurement:

Fifty (50) μl of the measuring solution and 50 μl of the test solution were mixed and left reacting at 37° C for one hour. The reaction mixture was cooled with ice to stop the reaction. Then 50 μl of allowed to be absorbed by a disk of DEAE cellulose paper 2.4 cm in diameter. The wet paper was washed three times with an aqueous 5% sodium disodium phosphate (with 12 molecules of crystal water) and once each with distilled water and ethanol. The radioactivity was determined with a liquid scintillation counter.
The results are shown in Table 2.

Table 2

| | Concentration (μg/ml) | Radio activity | | Average value (CPM) | % control |
|---|---|---|---|---|---|
| | | #1 | #2 | | |
| Positive control | | 7612 | 7448 | 7530.0 | |
| Negative control | | 364 | 423 | 393.5 | |
| Negative control | 200,000 | 970 | 930 | 967.0 | 92.0 |
| | 100,000 | 1231 | 1391 | 1391.0 | 86.0 |
| | 50,000 | 1828 | 3079 | 2450.5 | 71.1 |
| | 25,000 | 3143 | 4613 | 3878.0 | 51.2 |
| | 12,500 | 3666 | 4146 | 3906.0 | 50.8 |

It is clearly noted from Table 2 that Compound A manifested an activity to inhibit reverse transcriptase even in a small application rate.

This invention relates to an agent resistant to AIDS virus, having as an effective component thereof a salt of a heteropoly acid ion represented by the general formula:

$$[XM_{12}O_{40}]^{p-} \qquad (I)$$

This agent has an advantage that it acts very effectively on AIDS and inhibit or curb it and exhibits only inconspicuous toxicity.


## Claims

1. An agent resistant to AIDS virus, having as an effective component thereof a salt of a heteropoly acid ion represented by the following general formula I:

$$[XM_{12}O_{40}]^{p-} \qquad (I)$$

wherein X is an ion of one element selected from the group consisting of the elements of Groups III to VI in the Periodic Table of Elements and the transition metal elements, M is one ion or a mixture of up to 3 ions selected from the group consisting of the ions of molybdenum, tungsten, aluminum, vanadium, niobium, tantalum, cobalt, nickel, and titanium, O is an oxygen atom, and p is a positive integer.

2. An agent according to claim 1, wherein M represents a tungsten atom.

3. An agent according to claim 2, wherein said salt of a heteropoly acid ion is the salt of a cation selected from the group consisting of sodium, potassium, cesium, calcium, strontium, barium, ammonium, hydrogen, methyl ammonium, ethyl ammonium, dimethyl ammonium, trimethyl ammonium, tetramethyl ammonium, diethyl ammonium, isopropyl ammonium, diisopropyl ammonium, propyl ammonium, dipropyl ammonium, butyl ammonium, dibutyl ammonium, tributyl ammonium, tetrabutyl ammonium, and guanidinium.

4. An agent according to claim 3, wherein said salt of a heteropoly acid ion is at least one member selected from the group consisting of $K_5BW_{12}O_{40} \cdot 15H_2O$ and $K_7PW_{10}Ti_2O_{40} \cdot 6H_2O$.

5. A medicinal composition resistant to AIDS, comprising the salt of a heteropoly acid ion set forth in claim 1 and a pharmaceutically allowable carrier.

6. A composition according to claim 5, wherein M represents a tungsten atom.

7. A composition according to claim 6, wherein said salt of a heteropoly acid ion is the salt of a cation selected from the group consisting of sodium, potassium, cesium, calcium, strontium, barium, ammonium, hydrogen, methyl ammonium, ethyl ammonium, dimethyl ammonium, trimethyl ammonium, tetramethyl ammonium, diethyl ammonium, isopropyl ammonium, diisopropyl ammonium, propyl ammonium, dipropyl ammonium, butyl ammonium, dibutyl ammonium, tributyl ammonium, tetrabutyl ammonium, and guanidinium.

8. A composition according to claim 7, wherein said salt of a heteropoly acid ion is at least one member selected from the group consisting of $K_5BW_{12}O_{40} \cdot 5H_2O$ and $K_7PW_{10}Ti_2O_{40} \cdot 6H_2O$.

9. A composition according to claim 5, wherein said pharmaceutically allowable carrier is a solid or liquid carrier.

10. A composition according to claim 9, wherein said liquid carrier is water.

11. The use of the salt of a heteropoly acid ion set forth in claim 1 to 4 for the preparation of a medicinal composition useful for the treatment of AIDS.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-1 385 489 (ANVAR) * Page 2, right-hand column, lines 69-118; claims 1,6,9-11,18,25,26 * | 1-3,5-7 ,9-11 | A 61 K 33/42 A 61 K 33/24 |
| Y | * Page 2, right-hand column, lines 69-118 * | 4 | |
| X | FR-A-2 587 214 (INSTITUTE PASTEUR) * Claims 1,4; page 5, figure 1 * | 1-3,5-7 ,9-11 | |
| Y | INORG. CHEM., vol. 22, no. 5, 1983, pages 818-822, Washington, US; P.J. DOMAILLE et al.: "Ti2W10PO407-and [CpFe(CO)2Sn]2W10PO385-. Preparation, properties, and structure determination by tungsten-183 NMR" * Page 821, left-hand column: "Metal complexes of Ti2W10PO47-" * | 4 | |
| Y | J. CHEM. SOC. DALTON TRANS., no. 8, 1986, pages 1669-1675, London, GB; T. YAMASE et al.: "Photoredox chemistry of Keggin dodecatungstoborate [BW12040]5- and role of heterogeneous catalysis in hydrogen formation" * Page 1669: "Experimental" * | 1-11 | TECHNICAL FIELDS SEARCHED (Int. Cl.5) A 61 K |
| A | EP-A-0 185 584 (INSTITUTE PASTEUR) * Claims 1,3 * | 1-3,5-7 ,9-11 | |
| A | CHEMICAL ABSTRACTS, vol. 108, no. 12, 21st March 1988, page 446, abstract no. 101336t, Columbus, Ohio, US; JP-A-62 230 619 (POLYTRONICS K.K.) 09-10-1987 * Abstract * | 1-3,5-7 ,9-11 | |

The present search report has been drawn up for all claims

| Place of search | Date of completion of the search | Examiner |
|---|---|---|
| THE HAGUE | 08-05-1990 | BERTOCCHI C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)